Europäisches Patentamt

European Patent Office

Office européen des brevets

(11) Publication number: **0 216 558 B1**

## EUROPEAN PATENT SPECIFICATION

(45) Date of publication of patent specification: **08.07.92**

(51) Int. Cl.⁵: **A61K 33/30**, A61K 33/06, A61K 31/44, A61K 31/19, A61K 7/36, A61L 2/00, //(A61K33/30,33:06,31:44,31:19)

(21) Application number: **86306932.4**

(22) Date of filing: **09.09.86**

(54) Anti-microbial systems containing the magnesium sulfate adduct of 2,2'-dithiobis-pyridine-1,1'-dioxide and a water-soluble zinc salt.

(30) Priority: **11.09.85 US 774725**

(43) Date of publication of application:
**01.04.87 Bulletin 87/14**

(45) Publication of the grant of the patent:
**08.07.92 Bulletin 92/28**

(84) Designated Contracting States:
**DE FR GB IT**

(56) References cited:
**US-A- 3 890 434**
**US-A- 4 161 526**
**US-A- 4 235 873**

(73) Proprietor: **Chesebrough-Pond's Inc.**
**33 Benedict Place P.O. Box 6000**
**Greenwich, CT 06836(US)**

(72) Inventor: **Vishnupad, Mohan**
**79 Greenwood Lane**
**Monroe Connecticut 06468(US)**
Inventor: **Ramirez, Jose E.**
**15 Fox Court**
**Trumbull Connecticut 06611(US)**
Inventor: **Tanko, Robert J.**
**45 Riversview Court**
**Cheshire Connecticut 06480(US)**
Inventor: **Schmitt, William H.**
**103 Linden Avenue**
**Branford Connecticut 06405(US)**

(74) Representative: **Tonge, Robert James**
**UNILEVER PLC Patents Division P.O. Box 68**
**Unilever House**
**London EC4P 4BO(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to antimicrobial compositions containing the magnesium sulfate adduct of 2,2'-dithiobis-pyridine-1,1'-dioxide and a water-soluble zinc salt; more particularly, it relates to antimicrobial compositions containing a water-soluble, non-ionic pyrithione derivative known chemically as the magnesium sulfate adduct of 2,2'-dithiobis-pyridine-1,1'-dioxide (referred to herein as "the magnesium sulfate adduct") and a water-soluble zinc salt. More specifically, it has been found that the presence of a water-soluble zinc salt appears to enhance to an unexpected extent the anti-microbial activity of the magnesium sulfate adduct against certain types of micro-organisms, such as Pseudomonas aeruginosa.

US-A-4,161,526 discloses the prevention or removal of discoloration in pyrithione, pyrithione salt or dipyrithione compositions using a zinc salt. From US-A-3,890,434 are known hair shampoo compositions containing adducts of bis-(-2-pyridyl-1-oxide) disulfide which exhibit antimicrobial effects.

The antimicrobial compositions in accordance with the present invention may be incorporated in various useful therapeutic and cleansing compositions, such as surgical scrub compositions, skin disinfectants, mouthwashes, deodorants and hospital cleaners.

The magnesium sulfate adduct used in accordance with the present invention is a well known broad spectrum anti-microbial agent. For example, a well known commercially available form is sold by Olin Chemicals of Stanford, Conn., USA, under the trade name "OMADINE MDS", which is the trihydrate form.

While the magnesium sulfate adduct used in accordance with the present invention has broad spectrum antimicrobial activity, it lacks the desired activity against Pseudomonas aeruginosa, the microorganism associated with infection that often follows severe burning of the skin.

An object of the present invention is to provide antimicrobial composition wherein enhanced antimicrobial activity of the magnesium sulfate adduct is effectuated against certain types of microorganisms.

Another object of the present invention is to provide therapeutic and cleansing compositions having incorporated therein such an antimicrobial composition.

The present invention provides an antimicrobial composition characterised in that it comprises the magnesium sulfate adduct of 2,2'-dithiobis-pyridine-1,1'-dioxide and a water-soluble zinc salt in an amount having a synergistic effect against Pseudomonas areuginosa.

The preferred zinc salt used in accordance with the present invention is zinc chloride ($ZnCl_2$). Other water-soluble zinc salts which may be used include zinc acetate, zinc sulfate, zinc nitrate and zinc phenylsulfonate.

In general, it has been found that in order to obtain the desired enhancement of antimicrobial activity in accordance with the present invention the zinc salt should be in an amount of from 1 to 10 parts, by weight, per part of the magnesium sulfate adduct, preferably 1 part, by weight, per part of the magnesium sulfate adduct.

When the present composition is applied to a therapeutic and/or cleansing composition, the magnesium sulfate adduct in general is in an amount of from 0.1 to 1.5%, by weight, and the zinc salt from 0.1 to 1%, by weight, of the total composition.

It has been found that a most useful antimicrobial composition that may be obtained utilizing the present invention is a surgical scrub composition employing the present antimicrobial composition in an anhydrous foamable base composition. Such surgical scrub compositions have been found useful in killing the Pseudomonas aeruginosa bacterium. The anhydrous foamable base composition thereof contains petroleum jelly, mineral oil and a mild detergent (sodium cocoyl isethionate).

In order to illustrate the present invention by specific examples, a number of compositions containing the magnesium sulfate adduct and zinc chloride in accordance with the present invention were tested for antimicrobial activity and compared with control compositions containing either the magnesium sulfate adduct and/or zinc chloride. These compositions are reported in Table 1 with the antimicrobial activity thereof determined using the "Zone of inhibition test".

"Zone of inhibition test" is the relationship between a standard application of a test formulation on a solid agar surface and the resulting zone of inhibited growth of a test organism applied to the agar surface. The larger the zone of growth inhibition, the greater the antimicrobial activity. This test method is used to determine antimicrobial activity in both liquids and solids.

The compositions of Table 1 which exemplify of the present invention are:

2

TABLE 1

| SYNERGISTIC EFFECTS OF $ZnCl_2$ ON THE ACTIVITY OF THE MAGNESIUM SULFATE ADDUCT* vs. PSEUDOMONAS | | | |
|---|---|---|---|
| | ACTIVE INGREDIENTS AND PERCENTAGE | PHYSICAL FORM | ZONES (mm) vs. PSEUDOMONAS |
| Example A | Omadine MDS @ 0.135% | Solution | 0 |
| Example B | Aluminum chlorohydrate (ACH) @ 17.5% | Solution | 0 |
| Example C | Omadine MDS @ 0.135% ACH @ 17.5% | Solution | 0 |
| Example 1 | Omadine MDS @ 0.135% (ACH) @ 17.5% $ZnCl_2$ @ 0.1% | Solution | 7.6 |
| Example D | $ZnCl_2$ @ 0.1% | Lotion | 0 |
| Example E | Omadine MDS @ 0.135% ACH @ 17.5% | Solution | 0 |
| Example 2 | Omadine MDS @ 0.135% ACH @ 17.5% $ZnCl_2$ @ 0.1% | Solution | 8.6 |
| Example F | Omadine MDS @ 0.135% | Solution | 0 |
| Example 3 | Omadine MDS @ 0.135% $ZnCl_2$ @ 0.10% | Solution | 5.6 |
| Example G | $ZnCl_2$ @ 0.10% | Solution | 0 |
| Example H | Omadine MDS @ 0.135% | Solution | 0 |
| Example I | Omadine MDS @ 0.135% ACH @ 17.5% | Solution | 0 |
| Example 4 | Omadine MDS @ 0.135% ACH @ 17.5% $ZnCl_2$ @ 0.10% | Solution | 8.3 |
| Example J | Base Formula Only | Lotion | 0 |
| Example K | $ZnCl_2$ 1.0% | Lotion | 2.9 |
| Example L | Omadine MDS 0.225% | Lotion | 1.9 |
| Example 5 | Omadine MDS 0.225% $ZnCl_2$ 1.0% | Lotion | 11.9 |
| Example M | $ZnCl_2$ 2.0% | Solution | 1 |
| Example N | Omadine MDS 0.225 | Solution | 0.5 |
| Example 6 | Omadine MDs @ 0.5% $ZnCl_2$ 2.0% | Powder | 11.3 |

* The magnesium sulfate adduct is sold by Olin Chemicals under the trade name "OMADINE MDS".

Another useful determination for evaluating antimicrobial activity is to the minimal inhibitory concentration (M.I.C.) amount. The minimal inhibitory concentration is a serial two-fold dilution of the test formulation in a broth culture medium which is inoculated with a standardized culture of microorganisms. The amount of test agent that will inhibit visible microbial growth is termed the minimal inhibitory concentration (MIC) level. The lower the amount of test agent, the greater the antimicrobial activity.

The results of experimental analyses indicated an eight-fold increase in activity against Pseudomonas aeruginosa for the combination of $ZnCl_2$ with Omadine MDS compared to the activity of $ZnCl_2$ alone or Omadine MDS alone.

In still further comparison study, compositions were prepared and evaluated both by the minimum inhibitory concentration method and the zone of inhibition. The results were similar for all salts tested at 1% concentration with Omadine MDS at 0.5%. Again, the zone of inhibition was almost non-existent for controls 0-2 mm and 10-14 mm for the combination of the magnesium sulfate adduct and $ZnCl_2$ combination employed in accordance with the present invention.

In Table 2 there is reported a surgical scrub composition, Composition I, employing the above-mentioned anhydrous foaming base composition containing petroleum jelly, mineral oil, glycerine, $TiO_2$ and sodium cocoyl isethionate and the magnesium sulfate adduct and $ZnCl_2$ combination in accordance with the present invention and the control base Composition II.

3

# EP 0 216 558 B1

TABLE 2

| Formula | Composition I | Composition II |
|---|---|---|
| Petroleum jelly | 31.00 | 31.00 |
| Mineral oil | 19.50 | 19.50 |
| Glycerine | 5.00 | 5.00 |
| $TiO_2$ | 0.50 | 0.50 |
| Na cocoyl isethionate | 40.00 | 42.00 |
| Omadine MDS | 2.00 | 2.00 |
| $ZnCl_2$ (50% Solution) | 2.00 | - |

When tested for antimicrobial activity, the minimum inhibitory concentration activity was enhanced for the Omadine MDS 2%, $ZnCl_2$ 1% in Composition I, providing a substantial increase in activity compared to Composition II.

**Claims**

1. An antimicrobial composition characterised in that it comprises the magnesium sulfate adduct of 2,2'-dithiobis-pyridine-1,1'-dioxide and a water-soluble zinc salt in an amount having a synergistic effect against Pseudomonas areuginosa.

2. A composition as claimed in claim 1 wherein the zinc salt is selected from zinc chloride, zinc acetate, zinc sulfate, zinc nitrate and zinc phenylsulfonate.

3. A composition as claimed in claim 1 or claim 2 wherein the zinc salt is zinc chloride.

4. A composition as claimed in any of claims 1 to 3 wherein the zinc salt is in an amount of from 1 to 10 parts, by weight, per part of the antimicrobial adduct.

5. A composition as claimed in any of claims 1 to 4 wherein the magnesium sulfate adduct is in an amount of from 0.1 to 1.5%, by weight, and the zinc salt is in an amount of from 0.1 to 1%, by weight, of the total composition.

**Revendications**

1. Composition antimicrobienne, caractérisée en ce qu'elle comprend un produit d'addition de sulfate de magnésium avec le 1,1'-dioxyde de 2,2'-dithiobis-pyridine et un sel de zinc hydrosoluble en une quantité possédant un effet synergique contre Pseudomonas aeruginosa.

2. Composition selon la revendication 1, dans laquelle le sel de zinc est choisi parmi le chlorure de zinc, l'acétate de zinc, le sulfate de zinc, le nitrate de zinc et le phénylsulfonate de zinc.

3. Composition selon la revendication 1 ou 2, dans laquelle le sel de zinc est le chlorure de zinc.

4. Composition selon l'une quelconque des revendications 1 à 3, dans laquelle le sel de zinc est présent à raison de 1 à 10 parties en poids par partie du produit d'addition antimicrobien.

5. Composition selon l'une quelconque des revendications 1 à 4, dans laquelle le produit d'addition de sulfate de magnésium est présent à raison de 0,1 à 1,5% en poids et le sel de zinc est présent à raison de 0,1 à 1% en poids de la composition totale.

**Patentansprüche**

1. Antimikrobielle Zusammensetzung, dadurch gekennzeichnet, daß sie das Magnesiumsulfataddukt von 2,2'-Dithiobis-pyridin-1,1'-dioxid und ein wasserlösliches Zinksalz in einer Menge umfaßt, die einen synergistischen Effekt gegen Pseudomonas areuginosa aufweist.

4

**2.** Zusammensetzung nach Anspruch 1, worin das Zinksalz ausgewählt ist unter Zinkchlorid, Zinkacetat, Zinksulfat, Zinknitrat und Zinkphenylsulfonat.

**3.** Zusammensetzung nach Anspruch 1 oder Anspruch 2, worin das Zinksalz Zinkchlorid ist.

**4.** Zusammensetzung nach einem der Ansprüche 1 bis 3, worin das Zinksalz in einer Menge von 1 bis 10 Gewichtsteilen pro Teil des antimikrobiellen Addukts vorliegt.

**5.** Zusammensetzung nach einem der Ansprüche 1 bis 4, worin das Magnesiumsulfatadukt in einer Menge von 0,1 bis 1,5 Gew.-% und das Zinksalz in einer Menge von 0,1 bis 1 Gew.-% der Gesamtzusammensetzung vorliegt.